# FASCICULE DE BREVET EUROPEEN

(11) **EP 2 723 440 B1**
(45) Date de publication et mention de la délivrance du brevet: **03.06.2020**
(21) Numéro de dépôt: 12733631.1
(22) Date de dépôt: 22.06.2012
(51) Int. Cl.: A61M 39/06, A61M 39/00

(54) **DISPOSITIF DE VALVE HÉMOSTATIQUE DESTINÉ À L'INTRODUCTION D'UN MATÉRIEL MÉDICAL DANS UN PATIENT**
HEMOSTASIS VALVE DEVICE FOR INJECTING A MEDICAL MATERIAL INTO A PATIENT
HEMOSTASIS VALVE DEVICE FOR INJECTING A MEDICAL MATERIAL INTO A PATIENT

(30) Priorité: 22.06.2011 FR 1155495
(43) Date de publication de la demande: 30.04.2014
(73) Titulaire: Perouse Medical, 60173 Ivry le Temple (FR)
(72) Inventeur: PESSIN, Olivier, F-69290 Grezieu La Varenne (FR)
(74) Mandataire: Lavoix
(86) Numéro de dépôt international: PCT/EP2012/062127
(87) Numéro de publication internationale: WO 2012/175699

(56) Documents cités:
- WO-A1-00/53254
- WO-A1-03/048616
- US-A1- 2005 085 789
- US-A1- 2009 259 200

## Description

La présente invention concerne un dispositif de valve hémostatique selon le préambule de la revendication 1.

Un tel dispositif est destiné notamment à réaliser des interventions en cardiologie interventionnelle et en radiologie interventionnelle. Il est destiné en particulier à assurer l'étanchéité sanguine lors de l'introduction de dispositifs médicaux dans le corps d'un patient.

Le matériel médical est par exemple un cathéter, un fil guide, un guide, un cathéter à ballonnet serti ou non d'un stent, un outil rotatif de fraisage revêtu de microcristaux de diamants par exemple Rotablator® ou une combinaison de ces dispositifs.

Ce matériel médical est généralement introduit dans le corps du patient par l'intermédiaire du système vasculaire, notamment à travers une veine ou une artère, pour les amener jusqu'à la localisation précise de l'intervention dans le corps du patient.

Pour empêcher des fuites de fluide corporel, en particulier de sang, lors de l'introduction du matériel, il est nécessaire de placer un dispositif de valve hémostatique du type précité au niveau du point d'introduction du matériel dans le patient. US 5 324 271, WO 03/048616, US 2005/085789, WO 00/53254 et US 2009/259200 décrivent des exemples de dispositifs de valve hémostatique.

Ce dispositif comporte une valve proximale, destinée à réaliser une étanchéité autour du matériel lors de son introduction dans le corps. Il comporte en outre une valve distale, formée d'un manchon. Le manchon est compressible axialement par un organe d'actionnement vissable sur le corps.

La valve distale est actionnable une fois le matériel introduit à travers le passage interne. Pour réaliser l'étanchéité, le praticien doit visser l'organe d'actionnement, ce qui peut être fastidieux à réaliser.

En outre, la compression axiale de la valve permet de réaliser l'étanchéité autour du dispositif, mais ne garantit pas un maintien axial efficace du matériel médical dans le dispositif. Par ailleurs, le praticien ne peut pas déterminer simplement si le vissage qu'il a effectué est suffisant pour réaliser l'étanchéité autour du matériel, voire pour maintenir le matériel lui-même.

Un but de l'invention est donc d'obtenir un dispositif de valve hémostatique qui garantisse à la fois une bonne étanchéité tout en conservant la mobilité du matériel et, si besoin, à un moment précis de la procédure, un maintien efficace du matériel qu'il contient, tout en étant simple et rapide à utiliser.

A cet effet, l'invention a pour objet un dispositif selon la revendication 1.

Le dispositif selon l'invention peut comprendre l'une ou plusieurs des caractéristiques des revendications 2 à 15.

L'invention a également pour objet une méthode de traitement percutané comprenant les étapes suivantes :
- fourniture d'un dispositif tel que décrit ci-dessus, et d'un matériel médical destiné à être introduit dans le corps du patient ;
- avantageusement, actionnement de l'organe d'ouverture du passage pour libérer le passage et introduction du matériel dans le passage central de la valve ;
- actionnement de l'organe pour refermer le passage au travers de la valve
- extraction du matériel médical à travers l'ouverture distale et introduction dans le corps du patient ;
- actionnement de l'organe d'actionnement depuis sa première position vers sa deuxième position pour déplacer le ou chaque organe de compression de sa position rétractée à sa position déployée radicalement ;
- compression de l'organe compressible pour appliquer l'organe compressible autour du matériel en réduisant la section de la lumière de passage.

L'invention sera mieux comprise à la lecture de la description qui va suivre, donnée uniquement à titre d'exemple, et faite en se référant aux dessins annexés, sur lesquels :
- la figure 1 est une vue schématique de dessus d'un premier nécessaire de traitement selon l'invention ;
- la figure 2 est une vue en perspective éclatée du dispositif de valve hémostatique selon l'invention présent dans le nécessaire de la figure 1 ;
- la figure 3 est une vue de côté du dispositif de la figure 2 ;
- la figure 4 est une vue prise en coupe suivant un plan horizontal médian du dispositif de la figure 3 ;
- la figure 5 est une vue prise en coupe suivant un plan vertical médian du dispositif de la figure 3 ;
- la figure 6 est une vue analogue à la figure 4, l'organe d'ouverture de la valve élastique ayant été actionné ;
- la figure 7 est une vue analogue à la figure 4, l'organe d'actionnement de l'organe de compression ayant été actionné ;
- la figure 8 est une vue analogue à la figure 6 d'un deuxième dispositif de valve hémostatique selon l'invention ;
- la figure 9 est une vue analogue à la figure 7 du deuxième dispositif de valve selon l'invention ;
- le ou chaque organe de compression comporte une mâchoire présentant une surface concave destinée à s'appuyer sur une surface extérieure convexe de l'organe compressible,
- l'organe d'ouverture de la valve est monté mobile en translation sur l'organe d'actionnement, l'organe d'ouverture étant déplaçable conjointement avec l'organe d'actionnement lors de son déplacement entre la première position et sa deuxième position,
- la valve comporte un corps percé notamment un corps percé formé de deux disques percés assemblés l'un sur l'autre, le corps percé délimitant une pluralité de volets d'obturation, l'organe d'ouverture comprenant un manchon d'actionnement creux s'insérant entre les volets pour ouvrir le passage central de la valve,
- il comporte des moyens de blocage en position de l'organe d'actionnement dans sa deuxième position,
- la valve est disposée entre l'ouverture proximale du corps et l'organe compressible,
- l'organe d'actionnement est monté mobile en rotation par rapport au corps autour d'un axe D-D' perpendiculaire à l'axe longitudinal A-A' du corps,
- l'organe compressible est disposé axialement à l'écart de la valve d'étanchéité le long du corps creux.

L'invention a également pour objet un procédé de maintien axial d'un matériel médical, du type comprenant les étapes suivantes :
- fourniture d'un dispositif comme décrit plus haut, le matériel médical étant inséré à travers le passage interne d'introduction, l'organe compressible radialement occupant une configuration de repos dans laquelle la lumière de passage occupe une section transversale maximale ;
- déplacement de l'organe d'actionnement de sa première position à sa deuxième position ;
- déplacement radial du ou de chaque organe de compression radiale pour déformer radialement l'organe compressible et appliquer l'organe compressible ontre le matériel médical.

L'invention a également pour objet une méthode de traitement percutané comprenant les étapes suivantes :
- fourniture d'un dispositif tel que décrit ci-dessus, et d'un matériel médical destiné à être introduit dans le corps du patient ;
- avantageusement, actionnement de l'organe d'ouverture du passage pour libérer le passage et introduction du matériel dans le passage central de la valve ;
- actionnement de l'organe pour refermer le passage au travers de la valve
- extraction du matériel médical à travers l'ouverture distale et introduction dans le corps du patient ;
- actionnement de l'organe d'actionnement depuis sa première position vers sa deuxième position pour déplacer le ou chaque organe de compression de sa position rétractée à sa position déployée radicalement ;
- compression de l'organe compressible pour appliquer l'organe compressible autour du matériel en réduisant la section de la lumière de passage.

L'invention sera mieux comprise à la lecture de la description qui va suivre, donnée uniquement à titre d'exemple, et faite en se référant aux dessins annexés, sur lesquels :

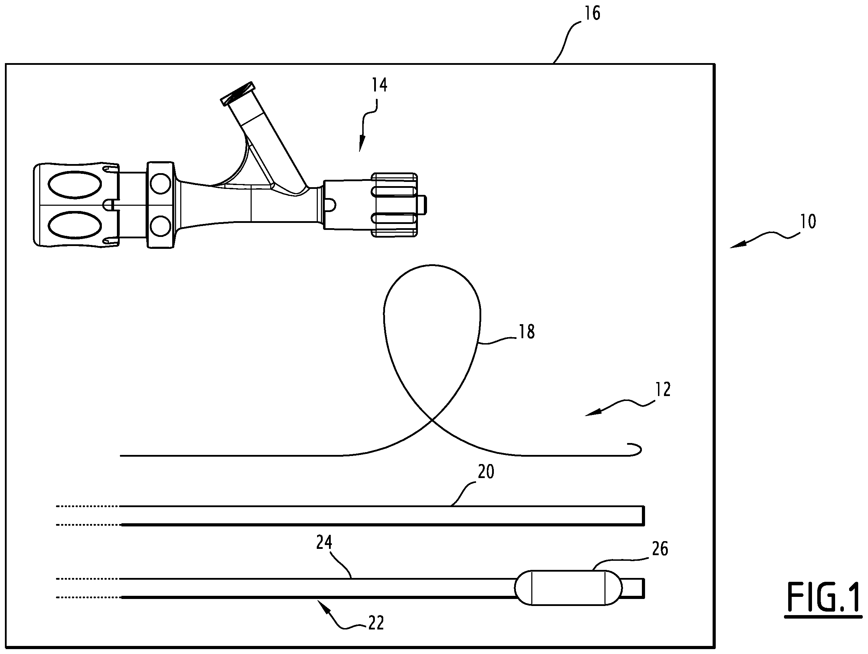

- la figure 1 est une vue schématique de dessus d'un premier nécessaire de traitement selon l'invention ;

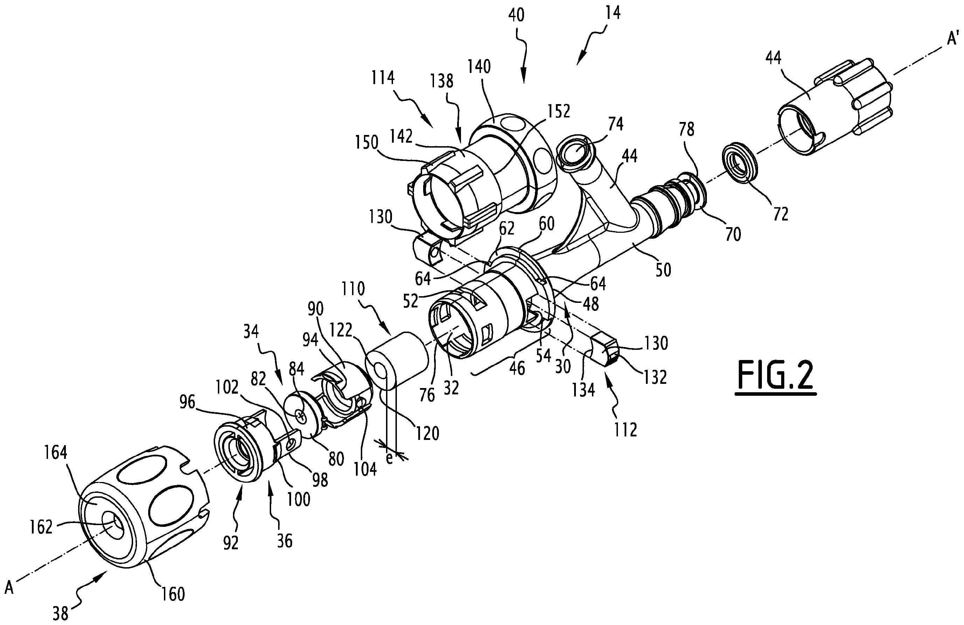

- la figure 2 est une vue en perspective éclatée du dispositif de valve hémostatique selon l'invention présent dans le nécessaire de la figure 1 ;

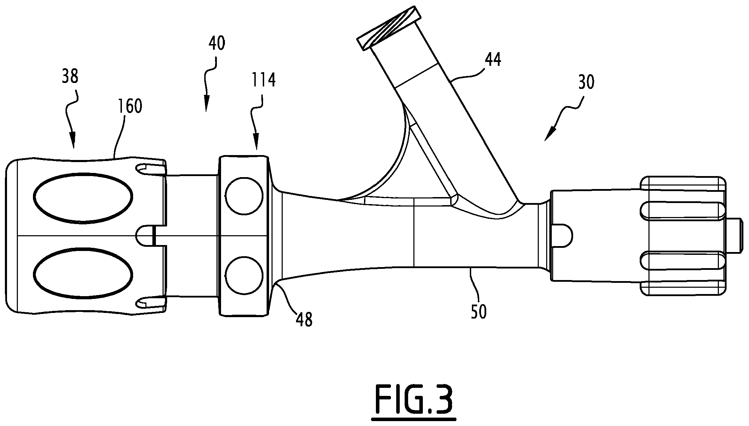

- la figure 3 est une vue de côté du dispositif de la figure 2 ;

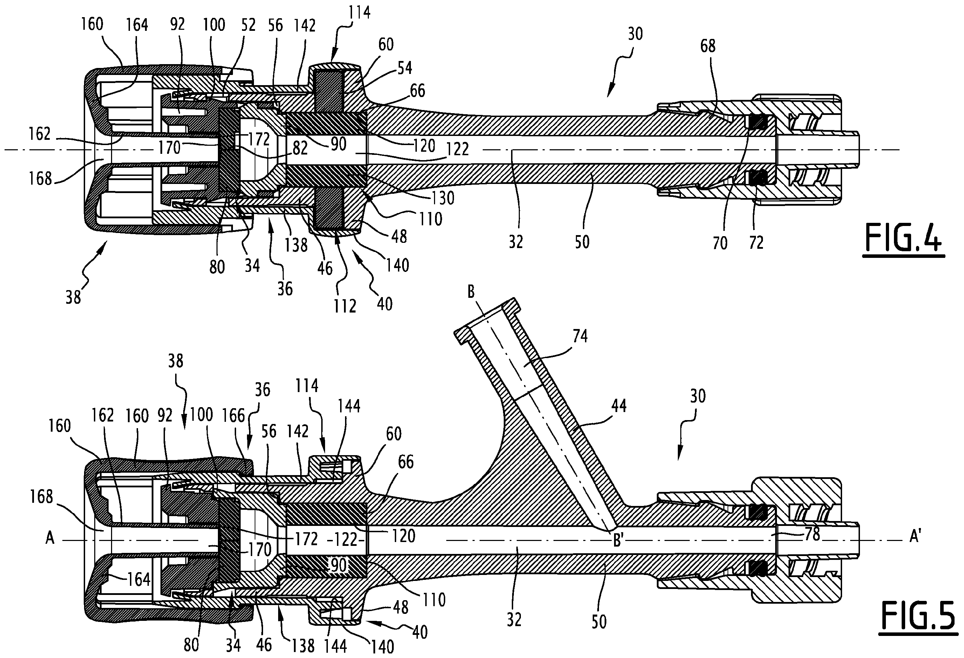

- la figure 4 est une vue prise en coupe suivant un plan horizontal médian du dispositif de la figure 3 ;
- la figure 5 est une vue prise en coupe suivant un plan vertical médian du dispositif de la figure 3 ;

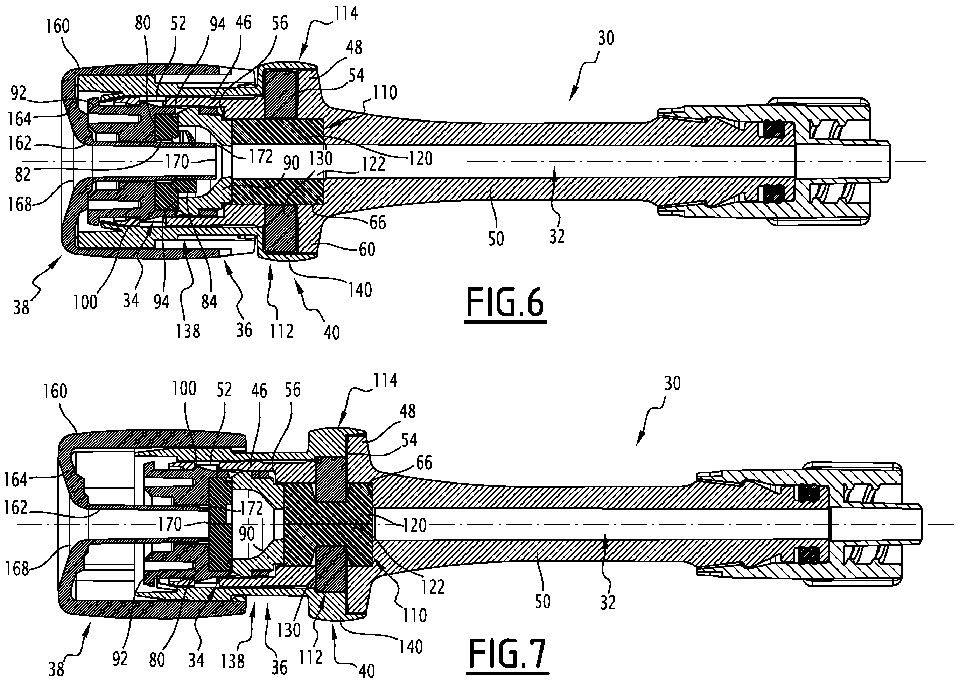

- la figure 6 est une vue analogue à la figure 4, l'organe d'ouverture de la valve élastique ayant été actionné ;
- la figure 7 est une vue analogue à la figure 4, l'organe d'actionnement de l'organe de compression ayant été actionné ;

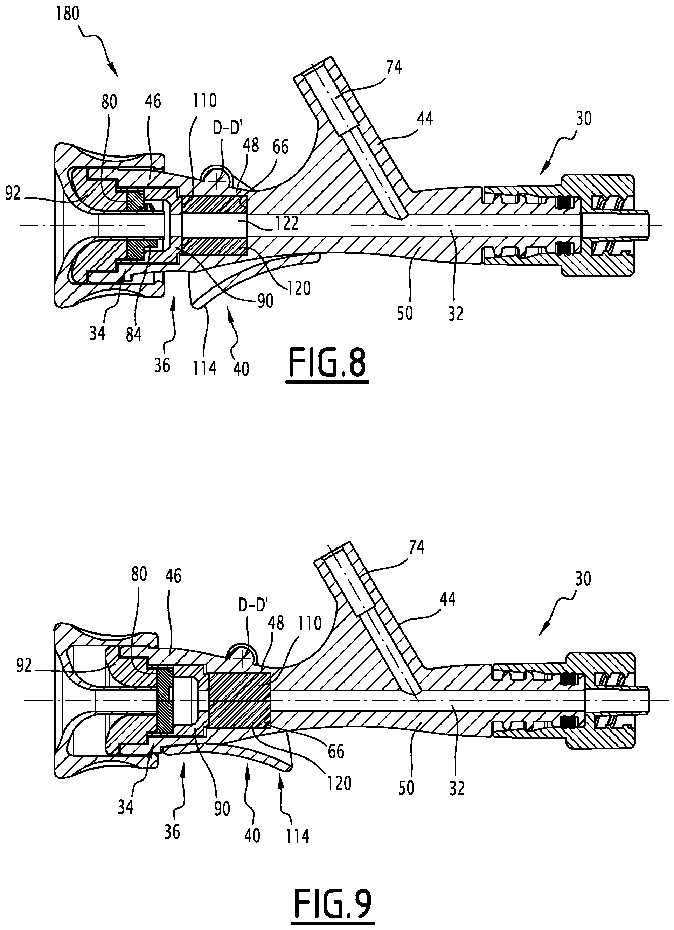

- la figure 8 est une vue analogue à la figure 6 d'un deuxième dispositif de valve hémostatique selon l'invention ;
- la figure 9 est une vue analogue à la figure 7 du deuxième dispositif de valve selon l'invention ;

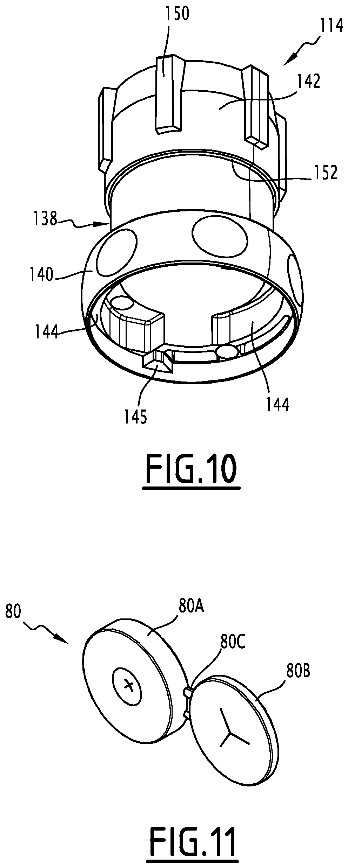

- la figure 10 est une vue en perspective de dessous de l'organe d'actionnement de l'organe de compression ; et
- la figure 11 est une vue en perspective d'un mode de réalisation de la valve avant sa mise en place dans le dispositif.

Dans tout ce qui suit, les termes « proximal » et « distal » s'entendent respectivement comme relativement plus près de l'opérateur, et comme relativement plus éloigné de l'opérateur.

Un premier nécessaire 10 selon l'invention est représenté sur la figure 1.

Comme illustré par la figure 1, le nécessaire 10 de traitement selon l'invention comporte au moins un matériel médical 12 destiné à être introduit dans le corps d'un patient, et un dispositif 14 de valve hémostatique selon l'invention, pour faciliter et contrôler l'introduction du matériel médical 12 dans le corps du patient.

Le nécessaire 10 selon l'invention est avantageusement contenu dans au moins un emballage stérile 16 représenté schématiquement sur la figure 1.

Le matériel 12 est destiné à être introduit dans le corps du patient, par l'intermédiaire d'une veine ou une artère.

Le matériel médical 12 destiné à être introduit dans le patient est notamment utilisé lors d'interventions dans le système vasculaire du patient, notamment en cardiologie interventionnelle et radiologie interventionnelle.

En particulier, dans l'exemple représenté sur la Figure 1, le matériel médical comprend un cathéter guide 20, un fil guide 18, un guide, un cathéter à ballonnet 22 serti ou non d'un stent, un Rotablator®

En fonction du matériel 12 introduit, et du nombre de dispositifs introduits simultanément, l'étendue radiale du matériel 12 est très variable.

Comme on le verra plus bas, le dispositif de valve hémostatique 14 est adapté pour accommoder du matériel 12 présentant des étendues radiales très différentes, par exemple, comprises entre 0,25 mm et 2 mm.

Comme illustré par les figures 2 et 3, le dispositif de valve hémostatique 14 selon l'invention comporte un corps creux 30 généralement tubulaire délimitant un passage interne 32 d'introduction du matériel.

Le dispositif 14 comporte en outre une valve d'étanchéité 34 élastique, de type « push-pull », et un mécanisme 36 de blocage axial de la valve 34.

Le dispositif 14 comprend aussi un organe 38 d'ouverture de la valve 34 et un ensemble 40 de maintien axial du matériel 12 dans le passage interne 32.

Comme illustré par les figures 2 à 4, le corps 30 comporte un élément tubulaire principal 42 et avantageusement, un piquage transversal 44 d'introduction d'un produit liquide dans le passage interne 32. Il est muni d'un embout 44, notamment d'un embout monté librement rotatif à l'extrémité distale de l'élément tubulaire 42.

En référence à la Figure 2, le corps 30 comporte une partie proximale 46 de logement de la valve élastique 34, une partie intermédiaire 48 de guidage de l'ensemble 40 de maintien axial, et une partie distale 50 portant l'embout 44.

La partie proximale 46 est de forme généralement cylindrique. Elle délimite des ouvertures proximales 52 de retenue du mécanisme de blocage 36 et des fenêtres distales 54 de passage des organes de compression de l'ensemble de maintien axial 40.

Les ouvertures proximales 52 et les fenêtres distales 54 traversent radialement la partie proximale 46 pour déboucher dans le passage interne 32.

Dans l'exemple représenté sur la figure 2, le corps 30 délimite deux fenêtres distales 54 disposées en regard l'une de l'autre, de part et d'autre d'un axe A-A' du corps.

En référence à la figure 4, la partie proximale 46 délimite avantageusement, dans le passage interne 32, un épaulement annulaire proximal 56 de blocage axial de la valve élastique 34.

La partie intermédiaire 48 comporte une collerette annulaire 60 faisant saillie radialement vers l'extérieur, pour le guidage de l'ensemble de maintien axial 40. La collerette 60 présente au moins une piste de guidage 62 délimitée angulairement par des butées de fin de course 64.

La partie intermédiaire 48 délimite, dans le passage interne 32, un épaulement annulaire distal 66 de retenue de l'ensemble de maintien axial 40. Les fenêtres distales 54 débouchent transversalement dans le passage interne 32 entre l'épaulement proximal 56 et l'épaulement distal 66.

À son extrémité distale, la partie distale 50 présente des éléments de retenue 68 de l'embout 44, et un logement annulaire 70 de réception d'un joint d'étanchéité 72 (voir figure 4).

L'embout rotatif 44 est destiné à être monté rotatif autour des éléments de retenue 68 à l'extrémité distale, avec interposition du joint 72 reçu dans le logement 70.

Comme illustré par la figure 5, le piquage transversal 44 fait saillie latéralement par rapport à l'axe A-A ' du corps 30, à partir de la partie distale 50 de l'élément tubulaire 42. Il présente un axe B-B' incliné d'un angle non nul par rapport à l'axe A-A'. L'angle formé par l'axe A-A ' du corps 30 et l'axe B-B' du piquage est par exemple compris entre 45° et 90°.

Le piquage transversal 44 délimite un passage auxiliaire 74 d'injection de liquide. Le passage 74 débouche en amont, à l'extérieur du dispositif 14 et en aval, dans le passage interne 32.

Le passage interne 32 s'étend entre une ouverture proximale 76 située à l'extrémité proximale de l'élément tubulaire 42, et une ouverture distale 78, située au niveau de l'embout 44.

Le diamètre du passage 32 dans la partie proximale 46, en amont de l'épaulement de blocage 56, est supérieur au diamètre du passage 32 dans la partie intermédiaire 48, entre le premier épaulement de blocage 56 et l'épaulement distal 66. Le diamètre du passage 32 dans la partie distale 50 est inférieur au diamètre du passage 32 dans la partie intermédiaire 48.

Comme illustré par les figures 2, 3 et 6, la valve élastique 34 est formée par un corps percé 80. Le corps percé 80 est fendu en son centre pour délimiter un passage central 82, obturé au repos.

Comme illustré par la Figure 11, le corps percé 80 est formé par un assemblage de deux disques 80A, 80B, avantageusement raccordés entre eux par une charnière 80C. Les disques 80A, 80B sont par exemple venus de matière. Le disque proximal 80A présente une épaisseur supérieure à celle du disque distal 80B. Le disque distal 80B est maintenu plaqué contre le disque proximal 80A par le mécanisme de blocage axial 36.

Le corps percé 80 comporte ainsi une pluralité de volets 84, mobiles élastiquement entre une position d'obturation du passage central 82, dans laquelle ils sont déployés transversalement par rapport à l'axe du corps percé 80, et une position contractée de libération du passage central 82.

La position d'obturation des volets 84 forme leur configuration de repos. La valve 34 est donc apte à revenir spontanément dans la position d'obturation lorsqu'elle n'est pas sollicitée.

Dans cet exemple, la valve élastique 34 s'étend transversalement au niveau de l'ouverture proximale 76 du passage 32. En variante, la valve 34 s'étend à l'intérieur du passage 32.

En référence aux figures 2 et 4, le mécanisme de blocage axial 36 comporte une entretoise 90 et une bague de blocage 92 placées de part et d'autre de la valve 34.

La valve 34 prend appui sur un anneau 94 de l'entretoise.

La bague de blocage 92 comporte un anneau proximal d'appui 96 sur la valve 34 et des organes de retenue 98 pour fixer axialement la bague de blocage 92, l'entretoise 90 et pour maintenir la valve 34 fixe entre ces éléments.

La bague 92 comporte des butées radiales extérieures 100 pour la fixer axialement sur le corps 30.

L'entretoise 90 est calée contre les ouvertures proximales 52 au moyen de deux butées radiales 100A.

Les organes de retenue 98 sont formés par des pattes. Les pattes 98 délimitent des logements 102 de réception d'une butée 104 solidaire de l'entretoise 90.

Ainsi, la valve 34 est maintenue serrée entre l'entretoise 90 et la bague de blocage 92. La bague de blocage 92 assure en outre le blocage axial par empilement mécanique de la valve 34, et de l'entretoise 90 sur le corps 30.

Dans une variante, le mécanisme de blocage axial 36 est dépourvu d'entretoise 90, la valve 34 étant appliquée directement sur l'épaulement 56.

Selon l'invention, l'ensemble de maintien 40 comporte un organe compressible 110 destiné à venir en prise avec le matériel introduit dans le passage 32 pour le retenir axialement. L'ensemble de maintien 40 comporte en outre au moins un organe 112 de compression radiale, déplaçable radialement par rapport à l'organe compressible 110, et au moins un organe d'actionnement 114 de chaque organe de compression radiale 112.

L'organe compressible 110 est formé dans cet exemple par un manchon 120 cylindrique délimitant une lumière 122 de passage du matériel. Le manchon 120 est avantageusement réalisé à base d'un matériau souple, par exemple d'un matériau présentant une dureté comprise entre 15 Shore A et 25 Shore A, notamment égale à 20 Shore A.

Pour accommoder différents matériels d'étendues radiales variées, le manchon 120 présente une épaisseur e1 radiale supérieure à l'étendue transversale de la lumière 122 qu'il délimite au repos. Ainsi, l'épaisseur e1 du manchon 120 est par exemple supérieure à 1,5 fois le demi diamètre de la lumière 122.

Dans le mode de réalisation représenté sur la figure 2, la lumière 122 est également cylindrique. Elle présente un diamètre supérieur à 2,3 mm.

L'épaisseur de la paroi du manchon 122 est par ailleurs supérieure à 2 mm.

La longueur du manchon 120, prise entre ses extrémités le long de l'axe A-A' est par exemple supérieure à 8 mm. Elle est notamment supérieure à 3 fois le diamètre de la lumière 122 au repos.

Comme on le verra plus bas, l'organe compressible 110 est déformable entre une configuration de repos sensiblement cylindrique, représentée sur les figures 1, 4, 5 et 6, dans laquelle la section transversale de la lumière 122 est maximale, et une configuration déformée radialement, représentée sur la figure 7, dans laquelle la section transversale de la lumière 122 est sensiblement nulle, au moins sur un tronçon axial de l'organe 110.

Dans cet exemple, l'organe compressible 110 est monté dans le passage 32 en étant calé axialement entre l'épaulement distal 66 et l'entretoise 90. Il s'étend en regard des fenêtres distales 54.

Dans l'exemple illustré par les figures 2, 4 et 7, l'ensemble de maintien axial 40 comporte une pluralité d'organes de compression radiale 112. En particulier, l'ensemble 40 comporte au moins deux organes 112 disposés de part et d'autre de l'axe A-A', en regard l'un de l'autre. Les organes 112 sont situés au contact d'une surface périphérique extérieure de l'organe compressible 110.

Chaque organe de compression radiale 112 est formé par une mâchoire 130 déplaçable radialement par rapport au corps 30, entre une position rétractée de repos, représentée sur la figure 4, et une position déployée radialement vers l'axe A-A', représentée sur la figure 7.

Chaque mâchoire 130 présente une surface radiale externe 132 destinée à coopérer avec l'organe d'actionnement 114, et une surface radiale interne 134 destinée à s'appliquer sur l'organe compressible 110.

Dans l'exemple représenté sur la figure 2, la surface radiale interne 134 de chaque mâchoire 130 est concave. La surface interne concave 134 présente ainsi une forme sensiblement complémentaire à la surface périphérique extérieure convexe du manchon 120 sur laquelle elle est appliquée.

La surface radiale extérieure 132 est avantageusement convexe.

Chaque mâchoire 130 est insérée à travers une fenêtre distale 54. Elle est guidée en translation radiale vers l'axe A-A' dans la fenêtre 54, entre la position rétractée de repos et la position déployée radialement.

Dans la position rétractée de repos, comme représenté sur la figure 4, chaque mâchoire 130 est disposée avantageusement à l'écart du passage 32. Elle est appliquée sur la surface périphérique extérieure de l'organe compressible 110, sans exercer de force significative sur cette surface. L'organe compressible 110 est donc maintenu dans sa configuration de repos.

Dans la position déployée, chaque mâchoire 130 s'est déployée radialement vers l'axe A-A' dans le passage interne 32. Elle comprime radialement vers l'axe A-A' l'organe compressible 110 pour produire un enserrement local du manchon 120 et une obturation de la lumière 122.

Dans cet exemple, l'organe d'actionnement 114 est monté mobile par rapport au corps 30, exclusivement en rotation autour de l'axe A-A', entre une première position de repos et une deuxième position d'actionnement de chaque organe de compression radiale 112.

L'organe d'actionnement 114 est formé généralement par un élément tubulaire 138. Il comporte une mollette 140 distale d'actionnement et, dans cet exemple, une partie proximale 142 de guidage et de blocage de l'organe d'ouverture de valve 38.

L'organe d'actionnement 114 présente intérieurement une came d'actionnement 144 de chaque organe de compression radiale 112.

L'élément tubulaire 138 est monté rotatif sur le corps 30 autour de l'axe A-A' en étant guidé par la collerette 60.

La mollette 140 fait saillie radialement par rapport à la partie proximale 142. Elle couvre extérieurement la collerette 60. Elle est munie d'orifices extérieurs de réception des doigts d'un utilisateur.

La molette 140 présente des butées complémentaires 145 visibles sur la Figure 10 destinées à coopérer avec les butées de fin de course 64 pour limiter la course de l'organe d'actionnement 114 entre la première position et la deuxième position.

Chaque came 144 présente une étendue radiale croissante vers l'axe A-A' en se déplaçant angulairement autour de l'axe A-A'. Elle est disposée au contact d'un organe de compression radiale 112.

Ainsi, chaque came 144 est propre à coopérer avec l'organe de compression 112 et à déplacer progressivement cet organe 112 radialement vers l'axe A-A' lors de la rotation de l'organe d'actionnement 114 autour de l'axe A-A'.

Dans cet exemple, la partie proximale 142 comprend des guides 150 de déplacement de l'organe d'ouverture 38 et une butée axiale 152 de retenue de l'organe 38. Les guides 150 s'étendent parallèlement à l'axe A-A'. Ils sont formés par des nervures longitudinales. La butée annulaire 152 est formée par un épaulement extérieur.

L'organe d'ouverture 38 de la valve 34 est ici monté coulissant sur l'organe d'actionnement 114. Il présente une paroi périphérique extérieure 160, un manchon interne 162 d'actionnement et une jupe 164 raccordant la paroi périphérique 160 au manchon 162 d'actionnement.

La paroi périphérique 160 est montée coulissante le long de l'axe A-A' par rapport au corps 30.

Dans cet exemple, la paroi 160 coiffe la partie proximale 142 de l'organe d'actionnement 114. Elle présente un rebord distal 166 de blocage destiné à coopérer axialement avec la butée de retenue 152.

Le manchon d'actionnement 162 s'étend coaxialement avec l'axe A-A' dans la paroi périphérique 160. Il débouche par une ouverture proximale 168 d'introduction du matériel 12 par une ouverture distale 170 située au niveau de son bord libre 172.

L'organe d'ouverture 38 est mobile en translation le long de l'axe A-A', entre une position proximale de repos et une position distale d'ouverture de la valve 34.

Dans la position proximale, visible sur la figure 5, le rebord 166 est disposé au contact de la butée de retenue 152 pour limiter le déplacement proximal de l'organe d'ouverture 38. Le bord distal 172 du manchon d'actionnement 162 est situé au contact de la valve 34, sans être inséré à l'intérieur du passage central 82.

Les volets 36 de la valve 34 occupent alors leur position déployée de repos. La valve 34 obture le passage 32.

Dans la position distale, visible sur la figure 6, l'organe d'ouverture 38 s'est déplacé le long de l'axe A-A' par rapport au corps 30 vers l'extrémité distale du corps 30.

Le rebord 166 s'est écarté de la butée 152 en se déplaçant vers l'extrémité distale du corps 30.

Dans cet exemple, le rebord 166 bute axialement contre la molette 140. La jupe 164 bute contre la bague 92.

Le manchon 162 s'est inséré à travers le passage central 82 de la valve 34 en écartant les volets 84 à l'écart de l'axe A-A'.

Un passage continu dégagé s'étend donc depuis l'ouverture proximale 168, à travers le manchon 162, jusqu'à l'ouverture distale 170 pour déboucher dans le passage 32.

Dans cette position, la valve 34 est ouverte et le matériel médical 12 peut être inséré à travers le dispositif 14.

Le fonctionnement du nécessaire 10 selon l'invention va maintenant être décrit.

Initialement, un cathéter guide (non représenté) est introduit dans le système vasculaire du patient.

Le dispositif 14 de valve hémostatique est alors connecté à une extrémité libre du cathéter guide par l'intermédiaire de l'embout rotatif 44.

De même, un ensemble d'injection de liquide, par exemple un set de coronarographie, est monté sur le piquage 44.

Ensuite, un débullage du dispositif 14 est effectué, pour éviter l'introduction d'air dans le système vasculaire.

Puis, le matériel médical 12 est introduit à travers le passage interne 32. A cet effet, l'opérateur actionne tout d'abord l'organe d'ouverture 38 pour le faire passer de sa position proximale à sa position distale.

Lors de ce passage, il insère le manchon 162 à travers le passage central de la valve 34 et dégage un passage continu pour l'introduction du matériel 12. Puis, il fait passer le matériel 12 à travers le manchon 162 et à travers le passage interne 32 du corps 30, pour atteindre l'ouverture distale 78.

Lorsque le matériel 12 a été introduit, l'opérateur ramène l'organe d'ouverture 38 dans sa position proximale, ce qui provoque le passage des volets 84 de la valve 34 vers leur position déployée par sollicitation élastique. Les volets 84 s'appliquent de manière étanche autour du matériel 12, assurant l'étanchéité vers l'extrémité distale du corps 30.

Ceci étant fait, l'opérateur peut alors modifier la position axiale du matériel médical 12 en tirant ou en poussant axialement le matériel 12 à travers la valve 34, sans risque de fuite.

Une fois le matériel 12 correctement positionné, l'opérateur manœuvre l'organe d'actionnement 114. Dans cet exemple, il l'entraîne en rotation autour de l'axe A-A', sans mouvement de translation. Ce déplacement est donc extrêmement simple et rapide à effectuer par rapport à un vissage.

Lors de ce déplacement, la came 144 pivote autour de l'axe A-A' et se déplace angulairement par rapport à chaque organe de compression radiale 112. L'organe de compression radiale 112 est donc poussé radialement vers l'axe A-A' par la came 144. Ceci provoque son application sur la surface extérieure de l'organe compressible 110 et la compression locale de l'organe compressible 110 vers la configuration déformée radialement.

La section de la lumière centrale 122 délimitée par le manchon 120 diminue donc en regard de chaque organe 112, jusqu'à ce que le manchon 120 s'applique autour du matériel médical 12 pour le maintenir axialement.

Le manchon 120 étant réalisé à base d'un matériau suffisamment déformable et suffisamment épais, l'opérateur poursuit donc le déplacement rotatif de l'organe d'actionnement 114 jusqu'à ce qu'il atteigne la fin de course, dans laquelle les butées de fin de course 64 entrent en contact avec les butées complémentaires présentes dans l'organe d'actionnement 114.

Compte tenu de l'épaisseur importante du manchon 120 et de la taille correspondante de la lumière 122, l'organe compressible 110 est apte à s'adapter à du matériel médical d'étendue radiale variée, et à fournir un serrage suffisant quelle que soit l'étendue radiale du matériel.

En outre, quelle que soit l'étendue radiale du matériel 12 introduit à travers la lumière 122, l'opérateur déplace l'organe d'actionnement 114 jusqu'à sa fin de course, ce qui garantit dans tous les cas une bonne tenue du matériel.

Puis, une intervention médicale peut être réalisée à l'aide du matériel 12.

Un deuxième nécessaire 180 selon l'invention est illustré par les figures 8 et 9.

A la différence du premier nécessaire 10, l'organe d'actionnement 114, est formé par un bouton 182 pivotant autour d'un axe D-D' perpendiculaire à l'axe A-A' du corps 30. Comme précédemment, aucun mouvement de vissage n'est nécessaire pour faire passer l'organe d'actionnement 114 de sa première position à sa deuxième position. Une simple rotation autour de l'axe D-D' suffit à effectuer ce passage.

Dans ce nécessaire 180, l'organe d'ouverture 38 est déplaçable indépendamment de l'organe d'actionnement 114. Il est ainsi monté mobile en translation directement sur le corps 30.

Comme sur le dispositif 14, il existe deux organes de compression 112 pour fermer et ouvrir la lumière 122. Le déplacement de ces organes 112 est commandé par le basculement de l'organe d'actionnement 114 qui forme un bouton à bascule.

Plus généralement, cette disposition indépendante de l'organe d'ouverture 38 peut s'appliquer aussi au dispositif décrit sur les figures 1 à 7.

Dans une variante, compte tenu de l'épaisseur du manchon 120, et de sa déformabilité, le dispositif 14 est apte à bloquer axialement une pluralité de matériels 12 montés en parallèle les uns par rapport aux autres dans le passage interne 32, par exemple un fil guide, un fil guide et un cathéter disposé parallèlement au fil guide, deux fils guides et un cathéter, deux fils guides et deux cathéter, ou encore un fil guide et une fraise de type Rotablator®

Dans une variante, le dispositif 14 comporte la présence des moyens de blocage de l'organe d'actionnement 114 dans sa deuxième position, en fin de course. Ceci facilite la manœuvre par l'opérateur, et garantit que l'opérateur effectue un serrage efficace du matériel médical 12.

Avantageusement, l'organe d'actionnement 114 de l'organe compressible 110 est sollicité vers la première position de repos dans toute position intermédiaire comprise entre la première position de repos et la deuxième position d'actionnement. Cette sollicitation résulte de la compression locale de l'organe compressible 110 par les mâchoires 130 dans la deuxième position.

Ainsi, les moyens de blocage en position maintiennent l'organe d'actionnement 114 dans la deuxième position à l'encontre de la sollicitation de l'organe d'actionnement 114 vers la première position.

La première position et la deuxième position de l'organe d'actionnement constituent les deux seules positions stables de l'organe d'actionnement 114, qui fonctionne à la manière d'un interrupteur.

Ainsi, lors du fonctionnement, si l'utilisateur débloque l'organe d'actionnement 114 en libérant les moyens de blocage, l'organe d'actionnement 114 sera amené spontanément vers la première position par déploiement de l'organe compressible 110 agissant sur les mâchoires 130 pour les déployer radialement, puis par coopération entre les mâchoires 130 et l'organe d'actionnement 114.

Lorsque l'organe d'actionnement est mobile en rotation autour de l'axe A-A', sa course angulaire autour de l'axe A-A' entre la première position et la deuxième position est généralement comprise entre 90° et 150°. Cette course est de préférence comprise entre 110° et 120° pour permettre un actionnement simple et rapide de l'organe 114.

Ceci optimise le déplacement des mâchoires 130 dans les fenêtres 54 entre leur position rétractée de repos et leur position déployée radialement vers l'axe longitudinal A-A'.

De préférence, et comme illustré sur la figure 2, chaque mâchoire 130 est mobile en translation radiale le long d'un axe perpendiculaire à l'axe A-A' coupant l'axe A-A'.

Les mâchoires 130 sont disjointes, et présentent une étendue angulaire maximale inférieure à 90°. Elles permettent donc d'appliquer une force radiale de compression très concentrée sur l'organe compressible radialement 110, avec une rotation minimale de l'organe d'actionnement 114.

Dans une autre variante, l'organe compressible radialement 110 est formé à partir d'un matériau présentant une dureté comprise entre 25 Shore A et 40 Shore A, avantageusement entre 26 Shore A et 35 Shore A.

## Revendications

1. Dispositif (14) de valve hémostatique destiné à l'introduction d'un matériel médical (12) dans un patient, du type comprenant :
- un corps creux (30) délimitant un passage interne (32) d'introduction du matériel (12) s'étendant entre une ouverture proximale (76) et une ouverture distale (78) ;
- une valve (34) d'étanchéité, disposée dans le passage interne (32), la valve d'étanchéité (34) présentant au moins un passage central (82) obturé au repos ;
- un organe (38) d'ouverture du passage central (82) de la valve (34), mobile par rapport au corps (30) entre une position de repos, dans laquelle la valve est obturée et une position d'ouverture de la valve (34) ;
- un ensemble (40) de maintien en position du matériel (12) dans le passage interne (32) du corps (30), l'ensemble (40) de maintien comportant :
- un organe compressible radialement (110) disposé dans le passage interne (32), l'organe compressible (110) délimitant au repos une lumière traversante (122) de passage du matériel (12) ;
- au moins un organe (112) de compression radiale de l'organe compressible (110), déplaçable radialement par rapport à un axe de la lumière de passage (122) pour comprimer extérieurement l'organe compressible (110) ;
- un organe (114) d'actionnement du ou de chaque organe de compression (112), mobile par rapport au corps (30) pour faire passer l'organe de compression (112) d'une position rétractée radialement, dans laquelle la section de la lumière de passage (122) est maximale, à une position déployée radialement dans l'organe compressible (110), dans laquelle la section de la lumière de passage (122) est minimale,
**caractérisé en ce que** chaque organe de compression radiale (112) est formé par une mâchoire (130) déplaçable radialement par rapport au corps (30) entre une position rétractée de repos et une position déployée radialement vers l'axe longitudinal (A-A') du corps (30), chaque mâchoire (130) étant insérée à travers une fenêtre distale (54) du corps (30) et étant guidée en translation radiale vers l'axe longitudinal (A-A') dans la fenêtre (54) entre la position rétractée de repos et la position déployée radialement, chaque mâchoire présentant une surface radiale externe destinée à coopérer avec l'organe d'actionnement, et une surface radiale interne destinée à s'appliquer sur l'organe compressible,
le dispositif comportant au moins deux organes de compression (112) opposés, l'organe d'actionnement (114) étant apte à déplacer simultanément les organes de compression (112) depuis leur position rétractée radialement vers leur position déployée radialement.

2. Dispositif (14) selon la revendication 1, **caractérisé en ce que** l'organe d'actionnement (114) est mobile en rotation autour d'un axe longitudinal A-A' du corps (30), la course angulaire de l'organe d'actionnement (114) autour de l'axe longitudinal (A-A') étant avantageusement comprise entre 90° et 150°, notamment entre 100° et 120°.

3. Dispositif (14) selon la revendication 2, **caractérisé en ce que** l'organe d'actionnement (114) est mobile exclusivement en rotation autour de l'axe longitudinal A-A' du corps (30), sans translation par rapport au corps (30).

4. Dispositif (14) selon l'une quelconque des revendications précédentes, **caractérisé en ce que** l'organe compressible (110) comporte un manchon élastique (120) délimitant au repos la lumière de passage (122), l'épaisseur du manchon (110), prise perpendiculairement à l'axe de la lumière de passage (122) étant supérieure à 50 % de l'étendue transversale maximale de la lumière de passage (122).

5. Dispositif (14) selon l'une quelconque des revendications précédentes, **caractérisé en ce que** l'organe compressible (110) est formé à partir d'un matériau présentant une dureté comprise entre 15 Shore A et 40 Shore A, avantageusement entre 15 Shore A et 25 Shore A ou entre 26 Shore A et 35 Shore A.

6. Dispositif (14) selon l'une quelconque des revendications précédentes, **caractérisé en ce que** le ou chaque organe de compression (112) comporte une mâchoire (130) présentant une surface concave (134) destinée à s'appuyer sur une surface extérieure convexe de l'organe compressible (110).

7. Dispositif (14) selon l'une quelconque des revendications précédentes, **caractérisé en ce que** l'organe d'actionnement (114) est monté mobile par rapport au corps (30) entre une première position de repos dans laquelle chaque organe de compression radiale (112) occupe sa position rétractée et une deuxième position d'actionnement dans laquelle chaque organe de compression radiale (112) occupe sa position déployée radialement.

8. Dispositif (14) selon la revendication 8, **caractérisé en ce que** l'organe d'ouverture (38) de la valve (34) est monté mobile en translation sur l'organe d'actionnement (114), l'organe d'ouverture (38) étant déplaçable conjointement avec l'organe d'actionnement (114) lors de son déplacement entre la première position et la deuxième position.

9. Dispositif (14) selon l'une quelconque des revendications 8 ou 9, **caractérisé en ce qu'**il comporte des moyens de blocage en position de l'organe d'actionnement (114) dans la deuxième position.

10. Dispositif (14) selon la revendication 10, **caractérisé en ce que** dans toute position intermédiaire comprise entre la première position et la deuxième position, l'organe d'actionnement (114) est sollicité vers la première position, les moyens de blocage en position maintenant l'organe d'actionnement (114) dans la deuxième position à l'encontre de la sollicitation de l'organe d'actionnement (114) vers la première position, la première position et la deuxième position constituant les deux seules positions stables de l'organe d'actionnement (114).

11. Dispositif (14) selon l'une quelconque des revendications précédentes, **caractérisé en ce que** la valve (34) comporte un corps percé (80) notamment un corps percé (80) formé de deux disques percés (80A, 80B) assemblés l'un sur l'autre, le corps percé (80) délimitant une pluralité de volets (84) d'obturation, l'organe d'ouverture (38) comprenant un manchon d'actionnement (162) creux s'insérant entre les volets (84) pour ouvrir le passage central de la valve (34).

12. Dispositif (14) selon l'une quelconque des revendications précédentes, **caractérisé en ce que** la valve (34) est disposée entre l'ouverture proximale (76) du corps (30) et l'organe compressible (110).

13. Dispositif (14) selon l'une quelconque des revendications précédentes, **caractérisé en ce que** l'organe d'actionnement (114) est monté mobile en rotation par rapport au corps (30) autour d'un axe (D-D') perpendiculaire à l'axe longitudinal (A-A') du corps.

14. Dispositif (14) selon l'une quelconque des revendications précédentes, **caractérisé en ce qu'**il comporte deux organes de compression (112) disposés de part et d'autre de l'axe longitudinal (A-A'), en regard l'un de l'autre, chaque organe de compression radiale (112) étant formé par une mâchoire (130), chaque mâchoire (130) étant insérée à travers une fenêtre distale (54), le corps (30) délimitant deux fenêtres distales (54) disposées en regard l'une de l'autre, de part et d'autre d'un axe longitudinal (A-A') du corps (30).

15. Dispositif (14) selon l'une quelconque des revendications précédentes, **caractérisé en ce que** chaque organe d'actionnement (114) est déplaçable en translation radiale suivant un axe perpendiculaire à l'axe longitudinal (A-A') entre la position rétractée et la position déployée.

## Patentansprüche

1. Hämostaseventilvorrichtung (14), die zur Einführung eines medizinischen Materials (12) in einen Patienten vorgesehen ist, des Typs, der umfasst:
- einen hohlen Körper (30), der einen sich zwischen einer proximalen Öffnung (76) und einer distalen Öffnung (78) erstreckenden Innendurchgang (32) zum Einführen des Materials (12) begrenzt;
- ein Abdichtungsventil (34), das in dem Innendurchgang (32) angeordnet ist, wobei das Abdichtungsventil (34) mindestens einen Mitteldurchgang (82) aufweist, der in der Ruhestellung verschlossen ist;
- ein Element (38) zum Öffnen des Mitteldurchganges (82) des Ventils (34), das in Bezug auf den Körper (30) zwischen einer Ruhestellung, in der das Ventil verschlossen ist, und einer Offenstellung des Ventils (34) bewegbar ist;
-- eine Anordnung (40) zum Halten des Materials (12) in Stellung in dem Innendurchgang (32) des Körpers (30), wobei die Anordnung (40) zum Halten aufweist:
-- ein radial komprimierbares Element (110), das in dem Innendurchgang (32) angeordnet ist, wobei das komprimierbare Element (110) in Ruhestellung ein durchgehendes Lumen (122) zum Durchführen des Materials (12) begrenzt;
- mindestens ein Element (112) für eine radiale Kompression des komprimierbaren Elementes (110), das in Bezug auf eine Achse des Durchführungslumens (122) radial bewegbar ist, um von außen das komprimierbare Element (110) zu komprimieren;
- ein Betätigungselement (114) des oder jedes Kompressionselements (112), das in Bezug auf den Körper (30) bewegbar ist, um das Kompressionselement (112) von einer radial zurückgezogenen Position, in der der Querschnitt des Durchführungslumens (122) maximal ist, in eine radial in das komprimierbare Element ausgefahrene Position (110) zu überführen, in der der Querschnitt des Durchführungslumens (122) minimal ist,
**dadurch gekennzeichnet, dass** jedes radiale Kompressionselement (112) von einer Spannbacke (130) gebildet wird, die radial in Bezug auf den Körper (30) zwischen einer zurückgezogenen Ruheposition und einer radial zur Längsachse (A-A') des Körpers (30) ausgefahrenen Position bewegbar ist, wobei jede Spannbacke durch ein distales Fenster (54) des Körpers (30) eingeführt wird und radial translatorisch zur Längsachse (A-A') in das Fenster (54) zwischen der zurückgezogenen Ruheposition und der radial ausgefahrenen Position geführt wird, wobei jede Spannbacke eine radiale Außenfläche, die vorgesehen ist, mit dem Betätigungselementes zusammenzuarbeiten, und eine radiale Innenfläche aufweist, die vorgesehen ist, sich an das komprimierbare Element anzulegen,
wobei die Vorrichtung mindestens zwei gegenüberliegende Kompressionselemente (112) aufweist, wobei das Betätigungselement (114) geeignet ist, die Kompressionselemente (112) gleichzeitig von ihrer radial zurückgezogenen Position in ihre radial ausgefahrene Position zu bringen.

2. Vorrichtung (14) nach Anspruch 1, **dadurch gekennzeichnet, dass** das Betätigungselement (114) um eine Längsachse A-A' des Körpers (30) drehbeweglich ist, wobei der Winkelweg des Betätigungselementes (114) um die Längsachse (A-A') vorteilhafterweise zwischen 90° und 150°, insbesondere zwischen 100° und 120° liegt.

3. Vorrichtung (14) nach Anspruch 2, **dadurch gekennzeichnet, dass** das Betätigungselement (114) nur bezüglich der Drehung um die Längsachse A-A' des Körpers (30) ohne Längsbewegung in Bezug auf den Körper (30) bewegbar ist.

4. Vorrichtung (14) nach einem beliebigen der vorhergehenden Ansprüche, **dadurch gekennzeichnet, dass** das komprimierbare Element (110) eine elastische Hülse (120) aufweist, die in der Ruhestellung das Durchführungslumen (122) begrenzt, wobei die Dicke der Hülse (110), senkrecht zur Achse des Durchführungslumens (122), größer als 50% der maximalen Querausdehnung des Durchführungslumens (122) ist.

5. Vorrichtung (14) nach einem beliebigen der vorhergehenden Ansprüche, **dadurch gekennzeichnet, dass** das komprimierbare Element (110) aus einem Material gebildet ist, das eine Härte zwischen 15 Shore A und 40 Shore A, vorzugsweise zwischen 15 Shore A und 25 Shore A oder zwischen 26 Shore A und 35 Shore A aufweist.

6. Vorrichtung (14) nach einem beliebigen der vorhergehenden Ansprüche, **dadurch gekennzeichnet, dass** das oder jedes Kompressionselement (112) eine Spannbacke (130) aufweist, die eine konkave Fläche (134) aufweist, die vorgesehen ist, sich an einer konvexen Außenfläche des komprimierbaren Elements (110) abzustützen.

7. Vorrichtung (14) nach einem beliebigen der vorhergehenden Ansprüche, **dadurch gekennzeichnet, dass** das Betätigungselement (114) in Bezug auf den Körper (30) zwischen einer ersten Ruhestellung, in der jedes radiale Kompressionselement (112) seine zurückgezogenen Position einnimmt, und einer zweiten Betätigungsstellung, in der jedes radiale Kompressionselement (112) seine radial ausgefahrene Position einnimmt, beweglich montiert ist.

8. Vorrichtung (14) nach Anspruch 8, **dadurch gekennzeichnet, dass** das Element (38) zum Öffnen des Ventils (34) translatorisch an dem Betätigungselement beweglich montiert ist, wobei das Öffnungselement (38) zusammen mit dem Betätigungselement (114) bei seiner Bewegung zwischen der ersten Stellung und der zweiten Stellung bewegbar ist.

9. Vorrichtung (14) nach einem beliebigen der Ansprüche 8 oder 9, **dadurch gekennzeichnet, dass** sie Mittel zum Sperren des Betätigungselementes (114) bezüglich der Position in der zweiten Stellung aufweist.

10. Vorrichtung (14) nach Anspruch 10, **dadurch gekennzeichnet, dass** in jeder Zwischenposition zwischen der ersten Stellung und der zweiten Stellung das Betätigungselement (114) in seine erste Stellung vorgespannt ist, wobei die Mittel zum Sperren bezüglich der Position das Betätigungselement (114) in seiner zweiten Stellung gegen die Vorspannung des Betätigungselementes (114) in seine erste Stellung hält, wobei die erste Stellung und die zweite Stellung die zwei einzigen stabilen Stellungen des Betätigungselementes (114) bilden.

11. Vorrichtung (14) nach einem beliebigen der vorhergehenden Ansprüche, **dadurch gekennzeichnet, dass** das Ventil (34) einen durchbohrten Körper (80), insbesondere einen durchbohrten Körper (80), der aus zwei durchbohrten und aneinander gesetzten Scheiben (80A, 80B) gebildet sind, aufweist, wobei der durchbohrte Körper (80) eine Mehrzahl von Verschlussflügeln (84) begrenzt, wobei das Öffnungselement (38) eine hohle Betätigungshülse (162) umfasst, die sich zwischen die Flügel (84) einführt, um den Mitteldurchgang des Ventils (34) zu öffnen.

12. Vorrichtung (14) nach einem beliebigen der vorhergehenden Ansprüche, **dadurch gekennzeichnet, dass** das Ventil (34) zwischen der proximalen Öffnung (66) des Körpers (30) und dem komprimierbaren Element (110) angeordnet ist.

13. Vorrichtung (14) nach einem beliebigen der vorhergehenden Ansprüche, **dadurch gekennzeichnet, dass** das Betätigungselement (114) in Bezug auf den Körper (30) um eine Achse (D-D') senkrecht zur Längsachse (A-A') des Körpers drehbeweglich montiert ist.

14. Vorrichtung (14) nach einem beliebigen der vorhergehenden Ansprüche, **dadurch gekennzeichnet, dass** sie zwei Kompressionselemente (112) aufweist, die beidseitig der Längsachse (A-A') einander gegenüberstehend angeordnet sind, wobei jedes radiale Kompressionselement (112) durch eine Spannbacke (130) gebildet ist und jede Spannbacke (130) durch ein distales Fenster (54) eingeführt wird, wobei der Körper (30) zwei distale Fenster (54) begrenzt, die beidseitig einer Längsachse (A-A') des Körpers (30) gegenüberstehend angeordnet sind.

15. Vorrichtung (14) nach einem beliebigen der vorhergehenden Ansprüche, **dadurch gekennzeichnet, dass** jedes Betätigungselement (114) radial translatorisch gemäß einer Achse senkrecht zur Längsachse (A-A') zwischen der zurückgezogenen Position und der ausgefahrenen Position bewegbar ist.

## Claims

1. A hemostasis valve device (14) intended for inserting a medical material (12) into a patient, of the type comprising:
- a hollow body (30) delimiting an inner passage (32) for inserting the material (12) extending between a proximal opening (76) and a distal opening (78);
- a sealing valve (34), positioned in the inner passage (32), the sealing valve (34) having at least one central passage (82) that is sealed when idle;
- a member (38) for opening the central passage (82) of the valve (34), movable relative to the body (30) between an idle position, in which the valve is sealed, and an open position of the valve (34);
- an assembly (40) for maintaining the material (12) in position in the inner passage (32) of the body (30), the maintaining assembly (40) including:
- a radially compressible member (110) positioned in the inner passage (32), the compressible member (110) delimiting, when idle, a through passage aperture (122) of the material (12);
- at least one radial compression member (112) for radially compressing the compressible member (110), radially movable relative to an axis of the passage aperture (122) to outwardly compress the compressible member (110);
- a member (114) for actuating the or each compression member (112), movable relative to the body (30) to cause the compression member (112) to go from a radially retracted position, in which the section of the passage aperture (122) is maximal, to a position that is radially deployed in the compressible member (110), in which the section of the passage aperture (122) is minimal.
**characterized in that** each radial compression member (112) is formed by a jaw (130) that is radially movable relative to the body (30) between an idle retracted position and a position that is deployed radially toward the longitudinal axis (A-A') of the body (30), each jaw (130) being inserted through a distal window (54) of the body (30) and being guided in radial translation toward the longitudinal axis (A-A') in the window (54) between the idle retracted position and the radially deployed position, each jaw having an external radial surface intended to cooperate with the actuating member, and an internal radial surface intended to be applied to the compressible member,
the device comprising at least two opposite compression members (112), the actuating member (114) being capable of simultaneously moving the compression members (112) from their radially retracted position toward their radially deployed position.

2. The device (14) according to claim 1, **characterized in that** the actuating member (114) is movable in rotation around a longitudinal axis A-A' of the body (30), the angular travel of the actuating member (114) around the longitudinal axis (A-A') advantageously being comprised between 90° and 150°, in particular between 100° and 120°.

3. The device (14) according to claim 2, **characterized in that** the actuating member (114) is movable only in rotation around the longitudinal axis A-A' of the body (30), without translation relative to the body (30).

4. The device (14) according to any one of the preceding claims, **characterized in that** the compressible member (110) includes an elastic sleeve (120) which, when idle, delimits the passage aperture (122), the thickness of the sleeve (110), considered perpendicular to the axis of the passage aperture (122), being greater than 50% of the maximal transverse expanse of the passage aperture (122).

5. The device (14) according to any one of the preceding claims, **characterized in that** the compressible member (110) is formed from a material having a hardness comprised between 15 Shore A and 40 Shore A, advantageously between 15 Shore A and 25 Shore A or between 26 Shore A and 35 Shore A.

6. The device (14) according to any one of the preceding claims, **characterized in that** the or each compression member (112) includes a jaw (130) having a concave surface (134) intended to bear on a convex outer surface of the compressible member (110).

7. The device (14) according to any one of the preceding claims, **characterized in that** the actuating member (114) is mounted movable relative to the body (30) between a first idle position, in which each radial compression member (112) is in its retracted position, and a second actuating position, in which each radial compression member (112) is in its radially deployed position.

8. The device (14) according to claim 8, **characterized in that** the opening member (38) of the valve (34) is mounted movable in translation on the actuating member (114), the opening member (38) being movable jointly with the actuating member (114) during its movement between the first position and the second position.

9. The device (14) according to any one of claims 8 or 9, **characterized in that** it includes means for locking the actuating member (114) in position in its second position.

10. The device (14) according to claim 10, **characterized in that** in any intermediate position comprised between the first position and the second position, the actuating member (114) is biased toward the first position, the means for locking in position maintaining the actuating member (114) in the second position against the bias of the actuating member (114) toward the first position, the first position and the second position constituting the only two stable positions of the actuating member (114).

11. The device (14) according to any one of the preceding claims, **characterized in that** the valve (34) includes a pierced body (80), in particular a pierced body (80) formed by two pierced discs (80A, 80B) assembled one on the other, the pierced body (80) delimiting a plurality of sealing shutters (84), the opening member (38) comprising a hollow actuating sleeve (162) inserted between the shutters (84) to open the central passage of the valve (34).

12. The device (14) according to any one of the preceding claims, **characterized in that** the valve (34) is positioned between the proximal opening (76) of the body (30) and the compressible member (110).

13. The device (14) according to any one of the preceding claims, **characterized in that** the actuating member (114) is mounted in rotation relative to the body (30) around an axis (D-D') perpendicular to the longitudinal axis (A-A') of the body.

14. The device (14) according to any one of the preceding claims, **characterized in that** it includes two compression members (112) positioned on either side of the longitudinal axis (A-A'), across from one another, each radial compression member (112) being formed by a jaw (130), each jaw (130) being inserted through a distal window (54), the body (30) delimiting two distal windows (54) positioned across from each other, on either side of a longitudinal axis (A-A') of the body (30).

15. The device (14) according to any one of the preceding claims, **characterized in that** each actuating member (114) is movable in radial translation along an axis perpendicular to the longitudinal axis (A-A') between the retracted position and the deployed position.
